# EUROPÄISCHE PATENTSCHRIFT

(11) **EP 3 621 943 B1**
(45) Veröffentlichungstag und Bekanntmachung des Hinweises auf die Patenterteilung: **18.01.2023**
(21) Anmeldenummer: 18717635.9
(22) Anmeldetag: 23.04.2018
(51) Int. Cl.: C07C 67/39, C07C 69/54

(54) **VERFAHREN FÜR OXIDATIVE VERESTERUNG VON ALDEHYDEN ZU CARBONSÄUREESTER**
METHOD FOR OXIDATIVE ESTERIFICATION OF ALDEHYDES TO CARBOXYLIC ACID ESTERS
PROCÉDÉ D'ESTÉRIFICATION OXYDATIVE D'ALDÉHYDES EN ESTERS D'ACIDE CARBONIQUE

(30) Priorität: 09.05.2017 EP 17170111
(43) Veröffentlichungstag der Anmeldung: 18.03.2020
(73) Patentinhaber: Röhm GmbH, 64295 Darmstadt (DE)
(72) Erfinder: LYGIN, Alexander, 64347 Griesheim (DE); KRILL, Steffen, 64367 Mühltal (DE); AIT AISSA, Belaid, 64287 Darmstadt (DE); ZSCHUNKE, Florian, 60433 Frankfurt (DE)
(74) Vertreter: Röhm Patent Association
(86) Internationale Anmeldenummer: PCT/EP2018/060255
(87) Internationale Veröffentlichungsnummer: WO 2018/206276

(56) Entgegenhaltungen:
- EP-A1- 2 886 529
- WO-A1-2014/170223
- US-B2- 8 450 235

## Beschreibung

### Gebiet der Erfindung

Die vorliegende Erfindung betrifft ein neuartiges Verfahren zur Durchführung einer heterogen-katalysierten Reaktion zur oxidativen Veresterung von Aldehyden zu Carbonsäureestern.

Vor diesem Hintergrund ist es mit dem vorliegenden erfindungsgemäßen Verfahren gelungen, solche Verfahren bei gleichbleibenden oder sogar gesteigerten Aktivitäten und Selektivitäten länger störungsfrei zu betreiben. Hieraus ergibt sich die Möglichkeit, solche Verfahren möglichst einfach, wirtschaftlich und umweltschonend durchzuführen.

### Stand der Technik

Die katalytische oxidative Veresterung von Aldehyden zur Herstellung von Carbonsäureestern ist in zahlreichen Patenten und Literaturstellen beschrieben. So kann man z.B. sehr effizient Methylmethacrylat aus Methacrolein und Methanol herstellen.

Wenn die leicht polymerisierbare Edukte und/oder Produkte eingesetzt bzw. hergestellt werden, dann ist es für einen wirtschaftlichen Prozess besonders wichtig die Polymerisation so weit wie möglich zu unterdrücken um die hohen Aktivitäten, Selektivitäten und Katalysatorstandzeiten zu erreichen. Besonders bei den teuren Edelmetall-haltigen Katalysator, welche z.B. auf Au, Pd, Ru oder Rh basieren, spielt die Katalysatorstandzeit eine entscheidende Rolle. Im Falle von oxidativen Veresterung von Methacrolein (MAL) zu Methylmethacrylat (MMA) ist es außerdem wünschenswert, dass die Reaktion in Gegenwart von relativ hohen MAL-Konzentrationen durchgeführt werden kann.

Im Stand der Technik wurde bisher nicht ausreichend beschrieben, wie man eine hohe Katalysatoraktivität, Selektivität und lange Standzeit ohne Deaktivierung und bei hohen MAL Konzentrationen im Reaktionsgemisch durchführen kann.

Das Verfahren für die direkte oxidative Veresterung von Methacrolein zu MMA ist vielfach beschrieben worden. So ist z.B. in der US 5,969,178 eine mit Pd-Pb katalysierte Umsetzung von MAL zu MMA mit einer Selektivität von 86,4 % bei einer Raum-Zeit-Ausbeute (RZA) von 5,5 mol MMA/kg Kat*h beschrieben. Dabei werden die möglichen MAL- und Methanol-Konzentrationen im Feed am Reaktoreingang ausführlich diskutiert, aber keine Informationen zur Zusammensetzung im Reaktor gegeben. Die Sauerstoffkonzentration im Reaktorabgas wird mit dem folgenden Hintergrund beschrieben und diskutiert: So soll diese im Abgas aufgrund der Explosionsgrenze kleiner als 8 Vol% sein. Weiterhin sei eine kleinere Sauerstoff-Konzentrationen im Reaktor, wie auch im Abgas, für die Reaktionsgeschwindigkeit nachteilig. So führten zu kleine Sauerstoff-Konzentrationen zur verstärkten Bildung von Nebenprodukten.

Auf der anderen Seite wird aber auch darauf hingewiesen, dass je größer die Sauerstoff-Konzentration ist, desto mehr Pb-Salze muss man dem Reaktor kontinuierlich zuführen, damit die Katalysatorperformance konstant und gut bleibt. Der aus allen diesen Gründen bevorzugte Einsatzbereich für einen Pd-Pb Katalysator liegt somit zwischen dem Sauerstoff-Partialdruck im Abgas von 0.98 kPa (0.01kg/cm²) und 78.4532 kPa (0.8 kg/cm²) bei zwischen 49.03325 kPa (0.5 kg/cm²) und 1961.33 kPa (20 kg/cm²) Gesamtdruck. In der besten Ausführungsform der US 5,969,178 des Ausführungsbeispiels 1 wird die Reaktion bei 294.1995 kPa (3 kg/cm²) Gesamtdruck und 9.31632 kPa (0.095 kg/cm²) O₂-Partialdruck im Abgas (entspricht 3,2 mol%) betrieben.

In der US 8,450,235 wurde der Einsatz eines NiO/Au-basierten Katalysators bei 0,5 MPa Gesamtdruck und 4 Vol% Sauerstoff im Abgas gezeigt. Die Selektivität zu MMA betrug 97,2%, die Raum-Zeit-Ausbeute 9,57 mol MMA/kg Kat*h. Das molare Verhältnis Methanol zu Methacrolein im Feed betrug dabei 4,36 (mol/mol). Das berechnete entsprechende Verhältnis im Reaktor betrug 14,7 (mol/mol).

Wenn nach der oxidativen Veresterung eine destillative Abtrennung von Methanol und Methacrolein stattfinden soll, wie es z.B. in US 5,969,178 beschrieben ist, ist es energetisch vorteilhafter das molare Verhältnis von Methanol zu Methacrolein im Reaktor auf unter 10 (mol/mol) zu reduzieren. In diesem Fall kann mehr Methanol als leichtsiedendes Azeotrop aus Methanol und Methacrolein abgetrennt werden. Vorteilhafterweise wird dabei weniger MMA mitrecycliert. Das Methanol-MAL-Azeotrop hat gemäß US 5,969,178 einen Siedepunkt von 58°C und eine Zusammensetzung von Methanol zu MAL von 72,2 Gew% zu 27,7 Gew%. Dabei liegt das molare Verhältnis von Methanol zu MAL bei 5,7. EP2886529 A1 beschreibt ein weiteres kontinuierliches Verfahren zur oxidativen Veresterung von Methacrolein mit Methanol und Sauerstoff unter Verwendung eins goldhaltigen Katalysators, wobei der Restanteil an Sauerstoff im Abgas ca. 4 Vol-% beträgt, eine 30.9 %-ige Lösung von Methacrolein in Methanol eingesetzt wird und die Stationärkonzentration von Methacrolein kleiner als 15 Gew% ist.

Wie die nachstehenden Beispiele deutlich zeigen, versucht man die ähnlichen dem US8450235B2 Versuche (mit 4 vol% O2 im Abgas) bei geringerem als 10 MeOH/MAL Verhältnis durchzuführen, so verschlechtert sich innerhalb kürzer Zeit die Aktivität und Selektivität des eingesetzten Katalysators dramatisch.

Weiteres Problem bei der Durchführung der oxidativen Veresterung von Mal zu MMA besteht darin, dass die flüchtigen Edukte (MAL und MeOH) über den Abgas, auch bei intensiver Kühlung, teilweise verloren gehen, was die Gesamtausbeute minimiert. Die Reduzierung von O₂-Konzentration im Abgas hat somit auch einen zusätzlichen Vorteil von Reduzierung der Wertstoffverluste über das Abgas.

### Aufgabe

In Anbetracht des Standes der Technik ist es daher Aufgabe der vorliegenden Erfindung, ein technisch verbessertes Verfahren zur Durchführung einer heterogen-katalysierten oxidativen Veresterungsreaktion zur Verfügung zu stellen. Dieses neuartige Verfahren soll insbesondere mit weniger Nachteilen als herkömmliche Verfahren des Standes der Technik behaftet sein.

Insbesondere sollen Verfahren des Standes der Technik dergestalt verbessert werden, dass nur ein minimaler Katalysatorverbrauch erfolgt und dadurch eine lange Standzeit des eingesetzten heterogenen Katalysators bei gleichzeitig guter und nahezu konstanten Katalysatoraktivität, Selektivität und guter Durchmischung im Reaktor möglich ist.

Darüber hinaus soll das Verfahren beim Einsatz von leicht polymerisierbaren Edukten und Bildung solcher Produkten und/oder Nebenprodukten eine solche Reaktorausführung ermöglichen, dass diese nur eine maximal sehr geringe Polymerisation zulässt.

Weiterhin soll das Verfahren gegenüber dem Stand der Technik kostengünstig sein, insbesondere ohne größere Katalysatorverluste durch Abrieb oder Austrag durchführbar sein und mit weniger und kürzeren Unterbrechungen des Betriebes erfolgen können. Daneben soll das Verfahren insbesondere sicher zu betreiben sein. Dazu sollen explosive oder überreaktive Zusammensetzungen innerhalb des Verfahrens weitestgehend vermieden werden.

Ferner sollte das Verfahren mit relativ einfachen und kostengünstigen Anlagen durchgeführt werden können. Die Anlagen sollten demgemäß mit geringen Investitionskosten verbunden sein. Hierbei sollen die Anlagen einfach zu warten sein, geringe Unterhaltskosten verursachen und sicher zu betreiben sein.

Weitere nicht explizit genannte Aufgaben ergeben sich aus dem Gesamtzusammenhang der nachfolgenden Beschreibung und der Ansprüche.

### Lösung

Gelöst werden diese Aufgaben durch die Zurverfügungstellung eines neuartigen Verfahrens zur kontinuierlichen Durchführung einer - beispielsweise heterogen-katalysierten - Reaktion zur oxidativen Veresterung von Methacrolein mit einem Alkylalkohol und Sauerstoff zu einem Alkylmethacrylat in einem Reaktorsystem, bestehend aus einem oder mehreren Reaktoren, in Anwesenheit eines goldhaltigen Katalysators, wobei der Reaktor mindestens eine Gaszuführung und mindestens eine Abgasentnahme aufweist. Dieses neuartige Verfahren ist dabei dadurch gekennzeichnet, dass das molare Verhältnis zwischen den stationären Konzentrationen des Alkylalkohols und des Methacroleins in dem Reaktor, bzw. innerhalb der einzelnen Reaktoren des Reaktorsystems kleiner 15 zu 1, insbesondere kleiner 10 zu 1 ist,
dass die Sauerstoffkonzentration in der Gasphase innerhalb des Reaktors, bzw. innerhalb der einzelnen Reaktoren des Reaktorsystems an der Stelle der Abgasentnahme unterhalb der Explosionsgrenze des austretenden Gasgemischs oder kleiner 7 vol% ist,
dass das stationäre molare Verhältnis von Alkylalkohol zu Methacrolein im Reaktor, bzw. innerhalb der einzelnen Reaktoren des Reaktorsystems zum molaren Verhältnis der Stoffe im Feed im stationären Zustand zwischen 1,5 und 15 beträgt, und dass die Stationärkonzentration von Methacrolein im Reaktor, bzw. innerhalb der einzelnen Reaktoren des Reaktorsystems kleiner 21 Gew% beträgt.

Überraschenderweise hat sich durch dieses neuartige Verfahren gezeigt, dass die kontinuierliche Durchführung einer mit Gold-haltigen Katalysatoren katalysierten oxidativen Veresterung von Methacrolein zu einem Alkylmethacrylat, wie insbesondere zu MMA mit einem Verhältnis der Konzentrationen des Alkylalkohols zum Methacrolein (im Folgenden MAL) C_{MeOH}/C_{MAL} kleiner 10:1 (mol/mol) im Reaktionsgemisch für eine lange Zeit mit hohen Katalysatoraktivität, Selektivität und ohne technischen Störungen möglich ist, wenn man die Sauerstoffkonzentration im Abgas auf weniger als 4 vol% O₂ einstellt. Dabei erreicht man eine gute Katalysatorperformance, z.B. in Bezug auf Ausbeute, Selektivität und sehr hohe Standzeiten und verliert zusätzlich nur die minimale Menge an flüchtigen Wertstoffen wie dem Alkylalkohol, insbesondere Methanol, MAL oder andere.

Entscheidend für die Reaktion ist erfindungsgemäß der vergleichsweise geringe Anteil an Sauerstoff in der Gasphase im Reaktor, bzw. in den Reaktoren. Der Sauerstoffanteil in dieser Gasphase variiert jedoch in Abhängigkeit verschiedener Faktoren innerhalb des Reaktors, bzw. in den Reaktoren lokal. So ist die Konzentration naturgemäß an der Stelle der Zuleitung des sauerstoffhaltigen Gases, wie beispielsweise Luft, am höchsten, während an anderen Stellen des jeweiligen Reaktors, insbesondere wenn die Gasphase dort bereits längeren Kontakt zu den Edukten und dem Katalysator hatte, diese Konzentration naturgemäß geringer. Diese lokale Verteilung hängt dabei von Faktoren wie der genauen Reaktorarchitektur und dem genauen Durchströmungsprofil desselben ab. Auch spielt die Gesamtgröße der Grenzfläche zwischen flüssigen und gasförmigen Phasen in dem jeweiligen Reaktor genauso eine Rolle wie die Verweilzeit der Gasphase in dem kontinuierlich betriebenen Reaktor. Daher lassen sich die Gaskonzentrationen im einzelnen Reaktor eigentlich nur schlecht beschreiben. Eine Ausnahme bildet dabei jedoch die Abführung des Abgases. Dieser Wert ist für jeden Reaktor unabhängig von dessen Architektur, den Grenzflächen, der Verweilzeit, den eingestellten Partialdrücken, dem Gesamtgasvolumen im Reaktor oder dem Durchströmungsprofil eindeutig bestimmbar und im Allgemeinen ein sehr gutes Maß für die insgesamt eingesetzte Sauerstoffmenge in einem Prozess. In Abhängigkeit von dem Aufbau und dem Füllstand eines Reaktors mit Fest- und Flüssigphasen kann der Fachmann zudem diesen Wert einfach durch die Einstellung des Innendrucks und die Zufuhrgeschwindigkeit des sauerstoffhaltigen Gases einstellen. Die Konzentration des Sauerstoffs kann dann im Reaktor direkt an der Entnahmestelle des Sauerstoffs mit dafür bekannten Sensoren festgestellt werden. Alternativ ist es aber auch möglich, die Konzentration auf dem ersten Stück der Abgasleitung zu bestimmen, insofern dass hier der Gesamtdruck und die Temperatur nicht oder nur im für den Fachmann bekannten Maße für eine Gaszusammensetzungsbestimmung von den Bedingungen im Reaktor abweichen. Das Abgasgemisch ist nach der Entnahme aus dem Reaktor nicht mehr reaktiv und die Konzentrationsbestimmung kann entsprechend auch hier problemlos erfolgen.

Besonders bevorzugt beträgt die Sauerstoffkonzentration in der Gasphase innerhalb des Reaktors, bzw. in den Reaktoren an der Stelle der jeweiligen Abgasentnahme kleiner 4,5 vol%, ganz besonders bevorzugt kleiner 4 vol%.

Das stationäre molare Verhältnis von Alkylalkohol zu Methacrolein im Reaktor, bzw. in den Reaktoren beträgt bevorzugt zum molaren Verhältnis der Stoffe im Feed im stationären Zustand zwischen 1,8 und 15. Besonders bevorzugt liegt dieser Faktor zwischen 1,9 und 14, ganz besonders bevorzugt zwischen 2 und 13. Dieses Merkmal gibt die Umsatzrate im stationären Zustand der Reaktion wieder. Durch die gezielte Einstellung dieses Faktors kann die Ausbeute der Reaktion überraschend gut optimiert werden.

Die Stationärkonzentration von Methacrolein im Reaktor, bzw. in den Reaktoren ist bevorzugt kleiner 15 Gew%, besonders bevorzugt kleiner 12 Gew%. Man kommt bei der erfindungsgemäßen Fahrweise der Reaktion zu besonders optimalen Resultaten, wenn man die Methacrolein-Konzentration im stationären Zustand im Reaktor eher geringer hält.

Es ist in einer Variante der Reaktion durchaus möglich, die Reaktion in zwei oder mehreren in Serie geschalteten Reaktoren durchzuführen. In diesem Fall beziehen sich die erfindungsgemäßen Merkmale, insbesondere Parameter auf den Feed vor dem jeweils ersten Reaktor und die innere Stationärkonzentration des jeweils letzten Reaktors.

Bevorzugt wird in dem erfindungsgemäßen Verfahren ein Katalysator verwendet, der in Form von Katalysatorpartikeln vorliegt. Diese Partikel weisen dabei bevorzugt die Elemente Sauerstoff, Silizium, Aluminium, mindestens ein basisches Element, Gold und optional, bevorzugt mindestens eines der Elemente Nickel, Kobalt Eisen und Zink auf. Bei den aufgeführten basischen Elementen kann es sich dabei um ein Alkalimetall, ein Erdalkalimetall, ein Seltenerdenmetall oder um Mischungen aus diesen Metallen handeln. Bei den basischen Elementen handelt es sich insbesondere um ein Alkalimetall (Li, Na, K, Rb, Cs, Fr), ein Erdalkalimetall (Be, Mg, Ca, Sr, Ba), ein Seltenerdenmetall (Sc, Y, La, Ce. Pr, Nd, Pm, Sm, Eu, Gd, Tb, Dy, Ho, Er, Tm, Yb, Lu) oder um Mischungen aus diesen Metallen. Das basische Element liegt dabei in der Regel auch als Oxid vor.

Ganz besonders bevorzugt besteht das Katalysatorpartikel ausschließlich aus Gold und den Oxiden des Siliziums, Aluminiums, Kobalts und mindestens einem der basischen Elemente. Ein Beispiel für eine besonders geeignete Zusammensetzung weist SiO₂, Al₂O₃, Co₃O₄, MgO und Au auf, insbesondere ausschließlich diese Verbindungen. Weiterhin sind die erfindungsgemäßen Katalysatorpartikel dadurch charakterisiert, dass die maximale Gold- bzw. die maximale Eisen-, Zink- oder Kobaltkonzentration des Katalysatorpartikels im Außenbereich desselben zu finden ist. Der besagte Außenbereich macht dabei maximal 60 %, bevorzugt maximal 40 % und besonders bevorzugt maximal 30 % des geometrischen Äquivalenzdurchmessers des Katalysatorpartikels aus. Dabei ist die Gold- bzw. Eisen-, Zink- und/oder Kobaltkonzentration in diesem Außenbereich mindestens 1,5 mal, bevorzugt mindestens zweimal und insbesondere bevorzugt mindestens 2,5 mal höher ist als die entsprechende Konzentration dieser Elemente im mittleren Bereich, der den verbliebenen Bereich des geometrischen Äquivalenzdurchmessers des Katalysatorpartikels ausmacht. Besonders bevorzugt befindet sich das Gold zu mehr als 90% in diesem Außenbereich.

Die Ermittlung und Analyse der Verteilung der Konzentrationen von Gold und/oder Eisen-, Zink- und/oder Kobalt entlang des Katalysatorpartikelprofils kann z.B. durch die Einbettung der Partikeln in eine Polymermatrix, nachfolgende Polierung und anschließende REM-EDX Analyse erfolgen. Eine analoge Analysemethode mittels Röntgen Microprobe (EPMA) wird z.B. in EP 2 210 664 A1 auf Seite 18 beschrieben.

Bevorzugt weisen die Katalysatorpartikel einen mittleren geometrischen Äquivalenzdurchmesser zwischen 1 und 1000 µm, bevorzugt zwischen 10 und 250 µm und besonders bevorzugt zwischen 25 und 200 µm auf. Die Dicke des Außenbereichs liegt dabei bevorzugt zwischen 2 und 100 µm, bevorzugt zwischen 5 und 50 µm. Die Größe des geometrischen Äquivalenzdurchmessers wird dabei angegeben, da die Partikel nicht zwingend gänzlich sphärisch vorliegen müssen, sondern durchaus auch komplexere Formen aufweisen können. Bevorzugt liegen die Partikel jedoch nahezu oder ideal-sphärisch vor.

Besonders bevorzugt liegen Gold und/oder Gold- und Metalloxid-, insbesondere Kobaltoxid-haltige Partikel mit einem mittleren Durchmesser zwischen 1 und 20 nm, bevorzugt 2 und 10 nm im Außenbereich des Katalysatorpartikels vor. Je nach Herstellungsverfahren ist es erfindungsgemäß sowohl möglich, dass das Gold in Form reiner Partikel als auch in einer Mischform z.B. mit dem Kobaltoxid vorliegt. Dabei ist das Gold in letzterem Fall in der Regel nur mit einem Teil des Kobaltoxids gemischt. Weiterhin ist es in beiden Ausführungsformen optional auch möglich, dass die Gold- bzw. goldhaltigen Partikel zur Stabilisierung zusätzlich mit einer dünnen Schicht, z.B. aus SiO₂ und/oder Al₂O₃ versehen werden.

Genauso bevorzugt sind die erfindungsgemäßen Katalysatorpartikel porös. Wobei sich die Porosität in der Regel nicht auf die Gold- bzw. goldhaltigen Phasen bezieht. Dabei weisen solche porösen Katalysatorpartikel eine spezifische Oberfläche zwischen 100 und 300 m²/g, bevorzugt zwischen 150 und 250 m²/g auf. Weiterhin beträgt in der Regel der durchschnittliche Porendurchmesser dabei 1 bis 50 nm, bevorzugt 2 bis 20 nm.

Bevorzugt liegt das Verhältnis der Katalysatormasse zum flüssigen Reaktionsmischvolumen im Reaktor zwischen 0,01 und 0,3 kg / L.

Bevorzugt liegt die Reaktionstemperatur zwischen 60 und 100 °C, besonders bevorzugt zwischen 70 und 95 °C. Der Reaktorinnendruck beträgt bevorzugt zwischen 1 und 20 bar, besonders bevorzugt zwischen 2 und 10 bar.

Das molare Verhältnis zwischen den stationären Konzentrationen des Alkylalkohols und des Methacroleins liegt besonders bevorzugt zwischen 4 zu 1 und 15 zu 1, insbesondere bevorzugt zwischen 4,5 zu 1 und 14 zu 1 und ganz besonders bevorzugt zwischen 5 zu 1 und 13 zu 1. Das molare Verhältnis zwischen den stationären Konzentrationen des Alkylalkohols und des Methacroleins liegt ganz besonders bevorzugt zwischen 4 zu 1 und 9,8 zu 1, und insbesondere ganz bevorzugt bevorzugt zwischen 4,5 zu 1 und 9,5 zu 1.

Besonders bevorzugt ist eine Ausführung des vorliegenden Verfahrens, bei dem der Sauerstoffpartialdruck im Reaktor, bzw. in den einzelnen Reaktoren an der Stelle der Abgasentnahme zwischen 0,01 und 0,8 bar und in der dem Reaktor zugeleiteten Gasmischung kleiner 5 bar beträgt.

Genauso bevorzugt, zusätzlich dazu oder unabhängig davon beträgt die stationäre Methacrolein-Konzentration im Reaktor, bzw. in den einzelnen Reaktoren zwischen 1 und 21 Gew%, besonders bevorzugt zwischen 3 und 21 Gew%, ganz besonders bevorzugt zwischen 5 und 20 Gew%.

Ganz besonders bevorzugt handelt es sich bei dem Alkylalkohol um Methanol und bei dem Alkylmethacrylat um Methylmethacrylat.

Als Reaktoren werden bevorzugt Slurryreaktoren eingesetzt. Im Falle einer Serienschaltung zweier oder mehrerer Reaktoren können auch verschiedenartige Reaktoren miteinander kombiniert werden.

Bevorzugt wird Reaktionsgemisch kontinuierlich aus dem Reaktor ausgetragen und der Katalysator verbleibt im Reaktor. In einer beispielhaften Ausführungsform wird dabei über mindestens einen, bevorzugt im jeweiligen Reaktor befindlichen Filter filtriert. Auch hier verbleibt der Katalysator nach der Filtration im Reaktor.

Alternativ wird das Reaktionsgemisch kontinuierlich aus dem Reaktor ausgetragen und über mindestens einen Filter, beispielsweise einen externen filtriert. Gleichfalls bevorzugt wird der Katalysator nach der Filtration zurück in den Reaktor geleitet. Dazu werden bevorzugt sich im Reaktor, besonders bevorzugt an der Peripherie im oberen Teil des Reaktors, befindende kontinuierlich betreibbare und optional zurückspülbare Filter eingesetzt. Danach wird der Katalysator nach der Filtration optional weiterbehandelt und teilweise oder vollständig zurück in den Reaktor geleitet. Bei dieser Weiterbehandlung kann es sich z.B. um ein Waschen, Reaktivieren oder eine Trennung nach Partikelgrößen handeln.

Die bevorzugt eingesetzte Filterporosität beträgt zwischen 5 und 100 Mikrometer, besonders bevorzugt zwischen 10 und 50 Mikrometer. Für einen zusätzlichen Rückhalt der feinen Katalysatorpartikel wird das einmal über Reaktorfilter filtrierte Reaktionsgemisch bevorzugt mindestens noch einmal über feinere Filter mit der Porosität von 1 bis 10 µm außerhalb vom Reaktor gefiltert, so dass die Partikel von maximal 5 µm zu mindestens 90% von dem Filter zurückgehalten werden.

Vor solche Filter kann bevorzugt, beispielsweise auch an der Peripherie des Reaktors, ein zusätzliches Absetzungssystem installiert werden. Dabei kann es sich um eine spezielle Zone mit laminarer Strömung, wo Sedimentation eines Großteils des eingesetzten Katalysators erfolgt, handeln. Eine solche Sedimentation erfolgt damit bevor es zur tatsächlichen Filtration kommt.

Vorteilhaft ist es, wenn das Reaktionsgemisch nach der kontinuierlichen Entnahme aus dem Reaktor in mindestens einer Destillationskolonne aufgearbeitet wird, und dabei der Alkylalkohol und Methacrolein als Destillat abgetrennt und zurück in den Reaktor geleitet werden.

Bevorzugt wird das für die Reaktion benötigte Gas über Gasverteiler, so genannte Sparger, im unteren Reaktorteil im feindispergiertem Zustand eindosiert. Bevorzugt wird das eingesetzte Gas in die Richtung zum Reaktorboden eindosiert, damit möglichst weniger Verstopfung mit den Katalysatorpartikeln auftreten kann.

Außer den für die Reaktion benötigten Edukten können verschiedene Hilfsstoffe wie z.B. Säuren, Basen, Polymerisationsinhibitoren, Antischaumbildner usw. dem Prozess zugeführt werden.

### Zeichnungen

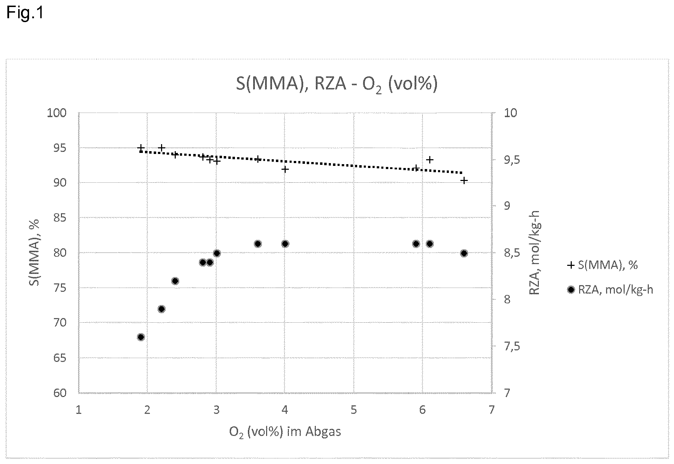

Bei Fig. 1 handelt es sich um die graphische Ausarbeitung der Versuchsergebnisse aus der weiter unten folgenden Tabelle 2. Insbesondere ist hier die Abhängigkeit der Raum-Zeit-Ausbeute bzw. der Selektivität der Reaktion von der O₂-Konzentration dargestellt.

### Beispiele

### Die Katalysatorherstellung

In einem 250 mL Becherglas werden 21,36 g Mg(NO₃)₂*6H₂O, 31,21 g Al(NO₃)₃*9H₂O zusammen vorgelegt und in 41,85 g VE Wasser unter Rühren mit einem Magnetrührer gelöst. Danach werden 1,57 g 60%ige HNO₃ unter Rühren zugegeben. 166,67 g Silicasol (Köstrosol 1530AS von der Firma Bad Köstritz, 30 Gew% SiO₂, Mittelgroße der Partikel: 15 nm) werden in einen 500 mL Dreihalskolben eingewogen und unter Rühren auf 15 °C gekühlt. 2,57 g 60%ige HNO₃ werden unter Rühren langsam zum Sol zugegeben. Bei 15 °C wird die Nitratlösung innerhalb von 45 min zum Sol unter Rühren zugegeben. Nach der Zugabe wird das Gemisch innerhalb von 30 min auf 50 °C erhitzt und weitere 24 h bei dieser Temperatur gerührt. Nach dieser Zeit wird das Gemisch bei 130 °C Ausgangstemperatur sprühgetrocknet. Das getrocknete Pulver (sphärisch, mittlere Partikelgroße 60 µm) wird in dünner Schicht im Naberofen innerhalb von 2h auf 300 °C erhitzt, 3 h bei 300 °C gehalten, innerhalb von 2 h auf 600 °C erhitzt und schließlich für 3 h bei 600 °C gehalten.

Eine Suspension von 10 g des SiO₂-Al₂O₃-MgO-Trägers aus dem vorherigen Abschnitt wird in 33,3 g VE Wasser auf 90 °C aufgeheizt und 15 min bei dieser Temperatur gerührt. Zu dieser Suspension wird unter Rühren eine vorher auf 90 °C erhitzte Lösung von HAuCl₄*3H₂O (205 mg) und Ni(NO₃)₂*6H₂O (567 mg, 1,95 mmol) in 8,3 g Wasser zugegeben. Nach der Zugabe wurde das Gemisch weitere 30 min gerührt, dann abgekühlt, bei Raumtemperatur filtriert und anschließend sechsmal mit jeweils 50 mL Wasser gewaschen. Das Material wurde 10 h bei 105 °C getrocknet, fein gemörsert, dann innerhalb von 1h von 18 auf 450 °C aufgeheizt und bei 450 °C für 5h kalziniert.

### Vergleichsbeispiele 1 bis 5

Gold-haltiger Katalysator (Menge siehe Tabelle 1) aus der vorherigen Beschreibung wurde in einem gerührten Druckreaktor mit einem Gas-einziehendem Rührer mit Reaktionsgemischvolumen von 200 mL bei 5 bar Druck kontinuierlich getestet. Dabei wurde kontinuierlich ein Feed aus 41 Gew% Methacrolein (im Weiteren: MAL) und 59 Gew% Methanol (44 g/h) sowie eine 0,5-1,0 Gew%ige NaOH-Lösung in Methanol (7,3 g/h) kontinuierlich in den Reaktor eingeleitet. Luft wurde als Sauerstoffquelle benutzt und in das flüssige Reaktionsgemisch direkt eindosiert. Das Abgas wurde nach dem Reaktor bei -20 °C abgekühlt und darin der Sauerstoffanteil kontinuierlich gemessen. Die in den Reaktor eingeleitete Luftmenge wurde so eingestellt, dass die Sauerstoffkonzentration im Abgas bei 4,0 vol% lag. Das flüssige Produktgemisch wurde nach der Filtration aus dem Reaktor kontinuierlich ausgetragen, abgekühlt und mittels Gaschromatographie (GC) analysiert.

**Tabelle 1**

| Bsp. | Masse Kat. [g] | MAL Konz. [wt%] | MeOH/MAL Im Reaktor [mol/mol] | U(MAL), [%] | S(MMA) [%] | RZA (max) [mol/kg kt-h] | TOS bis Deaktivierung >10% |
|---|---|---|---|---|---|---|---|
| VB1 | 5 | 22,3 | 5,6 | 35,3 | 86,0 | 16,1 | 100h |
| VB2 | 10 | 12,9 | 9,8 | 56,4 | 91,1 | 14,2 | 500h |
| VB3 | 15 | 10,0 | 12,2 | 66,2 | 94,8 | 10,6 | > 1000h |
| VB4 | 20 | 8,2 | 14,0 | 72,5 | 96,1 | 8,8 | > 1000h |
| VB5 | 25 | 6,9 | 16,6 | 76,7 | 96,0 | 7,5 | > 1000h |

| | | | | | | | |
|---|---|---|---|---|---|---|---|
| Bsp.: Beispiel; VB: Vergleichsbeispiel; Konz.: Konzentration; U: Umsatz; S: Selektivität; RZA: Raum-Zeit-Ausbeute; TOS: time on stream | | | | | | | |

Wie man sieht, lässt sich der Prozess bei der eingestellten Sauerstoff-Konzentration von 4 vol% nur dann mit einer guten Selektivität und hohen Standzeit durchführen, wenn das molare Verhältnis von Methanol zu MAL größer 10 ist.

### Vergleichsbeispiele 6 bis 16 (O₂ Anteil)

Gold-haltiger Katalysator (20 g) aus der vorherigen Beschreibung wurde wie in den Vergleichsbeispielen zuvor beschrieben getestet. Die einzige Variation war dabei die Einstellung der Sauerstoffkonzentration im Abgas, die zwischen 1,9 und 6,6 vol% variiert wurde. Weitere Prozessparameter und Ergebnisse sind in Tabelle 2 aufgelistet.

**Tabelle 2**

| Beispiel | O₂ im Abgas [mol%] | MAL Konz. Im Rkt. [wt%] | MeOH/MAL Im Reaktor [mol/mol] | U(MAL), [%] | S(MMA) [%] | RZA [mol/kg kt-h] |
|---|---|---|---|---|---|---|
| VB6 | 1,9 | 11,07 | 10,32 | 64,2 | 95,1 | 7,6 |
| VB7 | 2,2 | 10,44 | 10,94 | 65,9 | 95,1 | 7,9 |
| VB8 | 2,4 | 9,62 | 11,89 | 68,2 | 94,1 | 8,2 |
| VB9 | 2,8 | 8,59 | 12,97 | 71,2 | 93,8 | 8,4 |
| VB10 | 2,9 | 8,36 | 13,29 | 72,4 | 93,4 | 8,4 |
| VB11 | 3,0 | 9,01 | 12,52 | 70,1 | 93,2 | 8,5 |
| VB12 | 3,6 | 8,19 | 14,20 | 72,5 | 93,5 | 8,6 |
| VB13 | 4,0 | 8,29 | 14,04 | 72,2 | 92,0 | 8,6 |
| VB14 | 5,9 | 7,54 | 15,51 | 74,0 | 92,2 | 8,6 |
| VB15 | 6,1 | 8,13 | 14,49 | 72,2 | 93,4 | 8,6 |
| VB16 | 6,6 | 8,55 | 13,84 | 72,2 | 90,4 | 8,5 |

Anhand dieser Versuche kann man erkennen, dass bei relativ hohem Methanol-Anteil im Vergleich zur stationären MAL-Konzentration die Raum-Zeit-Ausbeute bis zu einer O₂-Konzentration von ca. 3 vol% zunimmt und darüber hinaus anscheinend nicht mehr von dieser abhängig ist. Die Selektivität dagegen scheint geringfügig mit steigender O₂-Konzentration abzunehmen. Dieser Zusammenhang ist zusätzlich in Fig. 1 abgebildet.

Bei O₂-Konzentration von über 6 vol% wurde insbesondere eine relativ schnelle Deaktivierung vom Katalysator und damit verbundene Reduzierung von RZA beobachtet. Insgesamt konnte festgestellt werden, dass eine steigende O₂-Konzentration einen negativen Einfluss auf die Katalysatorstandzeit hat.

Weiterhin kann man aus der Versuchsserie deutlich sehen, dass sich die Selektivität der Reaktion, S(MMA), mit steigender O₂-Abgaskonzentration reduziert. Der Grund für den Verlust der Selektivität und Katalysatordeaktivierung im Bereich von höheren O₂-Konzentrationen ist wahrscheinlich auf die Bildung von Oligo- und Polymeren des Methacroleins zurückzuführen.

Aus der Abhängigkeit der Raum-Zeit-Ausbeute RZA von der O₂-Konzentration kann man sehen, dass die Reaktion bei geringeren O₂-Anteilen durch Sauerstoffzufuhr limitiert ist, während ab einer bestimmten O₂-Konzentration (in diesem Fall zwischen 3 und 4 vol%) keine Abhängigkeit mehr ersichtlich ist., eine weitere Steigerung der O₂-Konzentration also keine Erhöhung der RZA mehr bringt.

Eine bevorzugte Durchführung der oxidativen Veresterung ist somit im Bereich der kinetischen Limitierung durch Sauerstoffzufuhr vorteilhaft, d.h. bei leichtem Unterschuss von O₂ wird der letzte schnell für die Reaktion aufgebraucht ohne die Nebenproduktenbildung (z.B. durch Polymerisation) zu erhöhen. Im Sauerstoffüberschuss (in diesem Fall bei über 4 vol% O₂ im Abgas) dagegen liegen höhere Mengen und Konzentrationen vom freien Sauerstoff im Reaktionsgemisch vor, was die Nebenproduktenbildung durch Polymerisation begünstigt.

### Beispiele 1 bis 10 und Vergleichsbeispiel 17 (Variation der Methanol/MAL Konzentrationen)

Gold-haltiger Katalysator (20 g) aus der vorherigen Beschreibung wurde analog zu den Vergleichsbeispielen zuvor, hier jedoch mit einer Sauerstoffkonzentration von 2,0 vol% im Abgas getestet. Eine konstante Menge an Feed aus MAL und Methanol (45 g/h, MAL Konzentration siehe in der Tabelle unten) und 0,5 bis 1 Gew%ige NaOH in Methanol (7,6 g/h) wurde in den Reaktor kontinuierlich eingeleitet. Ergebnisse sind in Tabelle 3 dargestellt.

**Tabelle 3**

| Bsp. | MAL im Feed [wt%] | MeOH im Feed [wt%] | MAL Konz. Im Rkt. [wt%] | MeOH/MAL Im Feed [mol/mol] | MeOH/MAL Im Reaktor [mol/mol] | Verhältnis Reaktor / FEED | U(MAL), [%] | S(MMA) [%] | RZA [mol/kg kt-h] |
|---|---|---|---|---|---|---|---|---|---|
| 10 | 40,06 | 59,94 | 11,07 | 3,27 | 10,32 | 3,15 | 64,2 | 94,3 | 7,62 |
| 1 | 41,54 | 58,46 | 11,65 | 3,08 | 9,48 | 3,08 | 63,5 | 94,3 | 7,83 |
| 2 | 41,24 | 58,76 | 12,39 | 3,12 | 8,84 | 2,84 | 61 | 94,5 | 7,43 |
| 3 | 41,85 | 58,15 | 12,64 | 3,04 | 8,70 | 2,86 | 60,7 | 94,4 | 7,53 |
| 4 | 42,81 | 57,19 | 13,98 | 2,92 | 7,79 | 2,67 | 57,8 | 94,3 | 7,41 |
| 5 | 44,2 | 55,80 | 16,34 | 2,76 | 6,33 | 2,29 | 53,7 | 94,6 | 7,05 |
| 6 | 44,7 | 55,30 | 16,51 | 2,71 | 6,46 | 2,39 | 53,2 | 94,8 | 7,09 |
| 7 | 47,76 | 52,24 | 20,1 | 2,39 | 5,09 | 2,13 | 48,1 | 94,4 | 6,91 |
| 8 | 47,74 | 52,26 | 19,88 | 2,39 | 5,09 | 2,13 | 48,2 | 92,8 | 6,70 |
| 9 | 47,85 | 52,15 | 20,48 | 2,38 | 4,98 | 2,09 | 46,8 | 91,4 | 6,36 |
| VB17 | 49,7 | 50,30 | 23,76 | 2,21 | 3,96 | 1,79 | 41,8 | 90,0 | 6,01 |

Das stationäre Verhältnis von Alkylalkohol zu Methacrolein im Reaktor zum Verhältnis der Stoffe im Feed im stationären Zustand beträgt in den in Tabelle 3 aufgeführten Versuchen zwischen 1,5 und 3,5.

Wie man sieht, ließ sich die Veresterungsreaktion überraschend problemlos und mit hoher Selektivität auch mit geringerem molarem Verhältnis von Methanol zu MAL betreiben, wenn man eine relativ geringe Sauerstoffkonzentration im Abgas (2 vol%) eingestellt hatte.

### Beispiele 11 und 12 (Langzeittest)

Gold-haltiger Katalysator (20 g) aus der vorangegangenen Beschreibung wurde in einem gerührten Druckreaktor mit einem Gas-einziehendem Rührer mit Reaktionsgemischvolumen von 200 mL bei 5 bar Druck kontinuierlich getestet. Dabei wurde kontinuierlich ein Feed aus 44,5 Gew% MAL und 55,5 Gew% Methanol (44g/h) sowie eine 1,0 Gew%ige NaOH-Lösung in Methanol (7,3 g/h) kontinuierlich in den Reaktor eingeleitet. Luft wurde als Sauerstoffquelle benutzt und in das flüssige Reaktionsgemisch direkt eindosiert. Das Abgas wurde nach dem Reaktor bei -20 °C abgekühlt und es wurde darin der Sauerstoffanteil kontinuierlich gemessen. Die in den Reaktor eingeleitete Luftmenge wurde so eingestellt, dass die Sauerstoffkonzentration im Abgas 2,0 vol% betrug. Das flüssige Produktgemisch wurde nach der Filtration aus dem Reaktor kontinuierlich ausgetragen, abgekühlt und mittels GC analysiert.

| Bsp. | Laufzeit [h] | MAL im Feed [wt%] | MAL Konz. Im Rkt. [wt%] | MeOH/MAL Im Reaktor [mol/mol] | U(MAL), [%] | S(MMA) [%] | RZA [mol/kg kt-h] |
|---|---|---|---|---|---|---|---|
| 11 | 500 | 44,5 | 16,5 | 6,5 | 53,2 | 94,8 | 7,09 |
| 12 | 2000 | 44,5 | 16,6 | 6,5 | 53,0 | 94,8 | 7,06 |

Wie man sieht, ließ sich die Veresterungsreaktion auch hier überraschend problemlos über eine längere Zeit mit hoher Selektivität auch mit geringerem molarem Verhältnis Methanol zu MAL betreiben, wenn man eine relativ geringe Sauerstoffkonzentration im Abgas eingestellt hatte.

## Patentansprüche

1. Verfahren zur kontinuierlichen Durchführung einer Reaktion zur oxidativen Veresterung von Methacrolein mit einem Alkylalkohol und Sauerstoff zu einem Alkylmethacrylat in einem Reaktorsystem, bestehend aus einem oder mehreren Reaktoren, in Anwesenheit eines goldhaltigen Katalysators, aufweisend mindestens eine Gaszuführung und mindestens eine Abgasentnahme, **dadurch gekennzeichnet, dass**
a. innerhalb der einzelnen Reaktoren des Reaktorsystems das molare Verhältnis zwischen den stationären Konzentrationen des Alkylalkohols und des Methacroleins kleiner 15 zu 1 ist,
b. die Sauerstoffkonzentration in der Gasphase innerhalb der einzelnen Reaktoren an der Stelle der Abgasentnahme unterhalb der Explosionsgrenze des austretenden Gasgemischs oder kleiner 7 vol% ist,
c. das stationäre Verhältnis von Alkylalkohol zu Methacrolein innerhalb der einzelnen Reaktoren zum molaren Verhältnis der Stoffe im Feed im stationären Zustand zwischen 1,5 und 15 beträgt und
d. die Stationärkonzentration von Methacrolein innerhalb der einzelnen Reaktoren kleiner 21 Gew% beträgt.

2. Verfahren gemäß Anspruch 1, **dadurch gekennzeichnet, dass**
a. innerhalb der einzelnen Reaktoren das molare Verhältnis zwischen den stationären Konzentrationen des Alkylalkohols und des Methacroleins zwischen 4 zu 1 und kleiner 10 zu 1 liegt,
b. die Sauerstoffkonzentration in der Gasphase innerhalb der einzelnen Reaktoren an der Stelle der Abgasentnahme unterhalb der Explosionsgrenze des austretenden Gasgemischs oder kleiner 4,5 vol% ist,
c. das stationäre Verhältnis von Alkylalkohol zu Methacrolein innerhalb der einzelnen Reaktoren zum Verhältnis der Stoffe im Feed im stationären Zustand zwischen 1,8 und 15 beträgt und
d. die Stationärkonzentration von Methacrolein innerhalb der einzelnen Reaktoren kleiner 15 Gew% beträgt.

3. Verfahren gemäß Anspruch 1, **dadurch gekennzeichnet, dass**
a. innerhalb der einzelnen Reaktoren das molare Verhältnis zwischen den stationären Konzentrationen des Alkylalkohols und des Methacroleins zwischen 5 zu 1 und kleiner 9,5 zu 1 liegt,
b. die Sauerstoffkonzentration in der Gasphase innerhalb der einzelnen Reaktoren an der Stelle der Abgasentnahme unterhalb der Explosionsgrenze des austretenden Gasgemischs oder kleiner 4 vol% ist,
c. das stationäre Verhältnis von Alkylalkohol zu Methacrolein innerhalb der einzelnen Reaktoren zum Verhältnis der Stoffe im Feed im stationären Zustand zwischen 2 und 13 beträgt und
d. die Stationärkonzentration von Methacrolein im Reaktor kleiner 12 Gew% beträgt.

4. Verfahren gemäß mindestens einem der Ansprüche 1 bis 3, **dadurch gekennzeichnet, dass** der Katalysator in Form von Katalysatorpartikeln, aufweisend die Elemente Sauerstoff, Silizium, Aluminium, mindestens ein basisches Element, Gold und optional eines der Elemente Nickel, Kobalt, Eisen und Zink, vorliegt, wobei es sich bei dem basischen Element um ein Alkalimetall, ein Erdalkalimetall, ein Seltenerdenmetall oder um Mischungen aus diesen Metallen handelt.

5. Verfahren gemäß mindestens einem der Ansprüche 1 bis 4, **dadurch gekennzeichnet, dass** die Reaktionstemperatur zwischen 60 und 100 °C und der Reaktorinnendruck zwischen 1 und 20 bar betragen.

6. Verfahren gemäß Anspruch 5, **dadurch gekennzeichnet, dass** die Reaktionstemperatur zwischen 70 und 95 °C und der Reaktorinnendruck zwischen 2 und 10 bar betragen.

7. Verfahren gemäß mindestens einem der Ansprüche 1 bis 6, **dadurch gekennzeichnet, dass** der Sauerstoffpartialdruck im Reaktor an der Stelle der Abgasentnahme zwischen 0,01 und 0,8 bar und in der dem Reaktor zugeleiteten Gasmischung kleiner 10 bar beträgt.

8. Verfahren gemäß mindestens einem der Ansprüche 1 bis 7, **dadurch gekennzeichnet, dass** die stationäre Methacrolein-Konzentration im Reaktor zwischen 3 und 21 Gew% beträgt.

9. Verfahren gemäß Anspruch 8, **dadurch gekennzeichnet, dass** die stationäre Methacrolein-Konzentration im Reaktor zwischen 5 und 20 Gew% beträgt.

10. Verfahren gemäß mindestens einem der Ansprüche 1 bis 9, **dadurch gekennzeichnet, dass** es sich bei dem Alkylalkohol um Methanol und bei dem Alkylmethacrylat um Methylmethacrylat handelt.

11. Verfahren gemäß mindestens einem der Ansprüche 1 bis 10, **dadurch gekennzeichnet, dass** das Verhältnis der Katalysatormasse zum flüssigen Reaktionsmischvolumen im Reaktor zwischen 0,01 und 0,3 kg / L beträgt.

12. Verfahren gemäß mindestens einem der Ansprüche 1 bis 11, **dadurch gekennzeichnet, dass** es sich bei dem Reaktor um einen Slurryreaktor handelt.

13. Verfahren gemäß mindestens einem der Ansprüche 1 bis 12, **dadurch gekennzeichnet, dass** Reaktionsgemisch kontinuierlich aus dem Reaktor ausgetragen wird und der Katalysator nach Abtrennung im Reaktor verbleibt.

14. Verfahren gemäß mindestens einem der Ansprüche 1 bis 13, **dadurch gekennzeichnet, dass** Reaktionsgemisch nach der kontinuierlichen Entnahme aus dem Reaktor in mindestens einer Destillationskolonne aufgearbeitet wird, und dabei der Alkylalkohol und Methacrolein als Destillat abgetrennt und zurück in den Reaktor geleitet werden.

## Claims

1. Process for continuously performing a reaction for oxidative esterification of methacrolein with an alkyl alcohol and oxygen to give an alkyl methacrylate in a reactor system consisting of one or more reactors, in the presence of a gold catalyst, having at least one gas feed and at least one offgas outlet, **characterized in that**
a. the molar ratio between the steady-state concentrations of the alkyl alcohol and of the methacrolein within the individual reactors of the reactor system is less than 15:1,
b. the oxygen concentration in the gas phase within the individual reactors at the site of the offgas outlet is below the explosion limit of the exiting gas mixture or less than 7% by volume,
c. the steady-state ratio of alkyl alcohol to methacrolein within the individual reactors to the molar ratio of the substances in the feed in the steady state is between 1.5 and 15 and
d. the steady-state concentration of methacrolein within the individual reactors is less than 21% by weight.

2. Process according to Claim 1, **characterized in that**
a. the molar ratio between the steady-state concentrations of the alkyl alcohol and of the methacrolein within the individual reactors is between 4:1 and less than 10:1,
b. the oxygen concentration in the gas phase within the individual reactors at the site of the offgas outlet is below the explosion limit of the exiting gas mixture or less than 4.5% by volume,
c. the steady-state ratio of alkyl alcohol to methacrolein within the individual reactors to the ratio of the substances in the feed in the steady state is between 1.8 and 15 and
d. the steady-state concentration of methacrolein within the individual reactors is less than 15% by weight.

3. Process according to Claim 1, **characterized in that**
a. the molar ratio between the steady-state concentrations of the alkyl alcohol and of the methacrolein within the individual reactors is between 5:1 and less than 9.5:1,
b. the oxygen concentration in the gas phase within the individual reactors at the site of the offgas outlet is below the explosion limit of the exiting gas mixture or less than 4% by volume,
c. the steady-state ratio of alkyl alcohol to methacrolein within the individual reactors to the ratio of the substances in the feed in the steady state is between 2 and 13 and
d. the steady-state concentration of methacrolein in the reactor is less than 12% by weight.

4. Process according to at least one of Claims 1 to 3, **characterized in that** the catalyst is in the form of catalyst particles comprising the elements oxygen, silicon, aluminium, at least one basic element, gold and optionally one of the elements nickel, cobalt, iron and zinc, wherein the basic element is an alkali metal, an alkaline earth metal, a rare earth metal or mixtures of these metals.

5. Process according to at least one of Claims 1 to 4, **characterized in that** the reaction temperature is between 60 and 100°C and the internal reactor pressure is between 1 and 20 bar.

6. Process according to Claim 5, **characterized in that** the reaction temperature is between 70 and 95°C and the internal reactor pressure is between 2 and 10 bar.

7. Process according to at least one of Claims 1 to 6, **characterized in that** the partial oxygen pressure in the reactor at the site of the offgas outlet is between 0.01 and 0.8 bar, and in the gas mixture fed to the reactor is less than 10 bar.

8. Process according to at least one of Claims 1 to 7, **characterized in that** the steady-state methacrolein concentration in the reactor is between 3% and 21% by weight.

9. Process according to Claim 8, **characterized in that** the steady-state methacrolein concentration in the reactor is between 5% and 20% by weight.

10. Process according to at least one of Claims 1 to 9, **characterized in that** the alkyl alcohol is methanol and the alkyl methacrylate is methyl methacrylate.

11. Process according to at least one of Claims 1 to 10, **characterized in that** the ratio of the mass of catalyst to the liquid reaction mixing volume in the reactor is between 0.01 and 0.3 kg/l.

12. Process according to at least one of Claims 1 to 11, **characterized in that** the reactor is a slurry reactor.

13. Process according to at least one of Claims 1 to 12, **characterized in that** reaction mixture is discharged continuously from the reactor and the catalyst, after being separated off, remains in the reactor.

14. Process according to at least one of Claims 1 to 13, **characterized in that** reaction mixture, after being withdrawn continuously from the reactor, is worked up in at least one distillation column, and the alkyl alcohol and methacrolein are separated off as distillate and returned to the reactor.

## Revendications

1. Procédé pour la mise en œuvre continue d'une réaction d'estérification oxydative de méthacroléine avec un alcool alkylique et de l'oxygène, pour obtenir un méthacrylate d'alkyle dans un système réactionnel constitué d'un ou plusieurs réacteurs, en présence d'un catalyseur contenant de l'or, comportant au moins une conduite d'amenée de gaz et un extracteur d'effluent gazeux, **caractérisé en ce que**
a. à l'intérieur des réacteurs individuels du système de réacteurs, le rapport en moles entre les concentrations stationnaires de l'alcool alkylique et de la méthacroléine est inférieur à 15 et va jusqu'à 1,
b. la concentration de l'oxygène dans la phase gazeuse à l'intérieur des réacteurs individuels au point d'extraction des effluents gazeux est inférieure à la limite explosive du mélange gazeux sortant, ou est inférieure à 7 % en volume,
c. le rapport stationnaire de l'alcool alkylique à la méthacroléine à l'intérieur des réacteurs individuels, par rapport au rapport en moles des matières dans la charge à l'état stationnaire, est compris entre 1,5 et 15 et
d. la concentration stationnaire de la méthacroléine à l'intérieur des réacteurs individuels est inférieure à 21 % en poids.

2. Procédé selon la revendication 1, **caractérisé en ce que**
a. à l'intérieur des réacteurs individuels, le rapport en moles entre les concentrations stationnaires de l'alcool alkylique et de la méthacroléine est compris entre 4:1 et inférieur à 10:1,
b. la concentration de l'oxygène dans la phase gazeuse à l'intérieur des réacteurs individuels au point d'extraction de l'effluent gazeux est inférieure à la limite explosive du mélange gazeux sortant ou est inférieure à 4,5 % en volume,
c. le rapport stationnaire de l'alcool alkylique à la méthacroléine à l'intérieur des réacteurs individuels par rapport au rapport des matières dans la charge à l'état stationnaire est compris entre 1,8 et 15, et
d. la concentration stationnaire de la méthacroléine à l'intérieur des réacteurs individuels est inférieure à 15 % en poids.

3. Procédé selon la revendication 1, **caractérisé en ce que**
a. à l'intérieur des réacteurs individuels, le rapport en moles entre les concentrations stationnaires de l'alcool alkylique et de la méthacroléine est compris entre 5:1 et inférieur à 9,5:1,
b. la concentration de l'oxygène dans la phase gazeuse à l'intérieur des réacteurs individuels au point d'extraction de l'effluent gazeux est inférieure à la limite explosive du mélange gazeux sortant ou est inférieure à 4 % en volume,
c. le rapport stationnaire de l'alcool alkylique à la méthacroléine à l'intérieur des réacteurs individuels par rapport au rapport des matières dans la charge à l'état stationnaire est compris entre 2 et 13, et
d. la concentration stationnaire de la méthacroléine dans le réacteur est inférieure à 12 % en poids.

4. Procédé selon au moins l'une des revendications 1 à 3, **caractérisé en ce que** le catalyseur se présente sous forme de particules de catalyseur, comportant les éléments oxygène, silicium, aluminium, au moins un élément basique, de l'or et éventuellement l'un des éléments nickel, cobalt, fer et zinc, l'élément basique étant un métal alcalin, un métal alcalino-terreux, un métal des terres rares ou des mélanges de ces métaux.

5. Procédé selon au moins l'une des revendications 1 à 4, **caractérisé en ce que** la température de la réaction est comprise entre 60 et 100 °C et la pression interne dans le réacteur est comprise entre 1 et 20 bar.

6. Procédé selon la revendication 5, **caractérisé en ce que** la température de la réaction est comprise entre 70 et 95 °C et la pression intérieure dans le réacteur est comprise entre 2 et 10 bar.

7. Procédé selon au moins l'une des revendications 1 à 6, **caractérisé en ce que** la pression partielle de l'oxygène dans le réacteur au point d'extraction de l'effluent gazeux est comprise entre 0,01 et 0,8 bar et, dans le mélange gazeux amené au réacteur, est inférieure à 10 bar.

8. Procédé selon au moins l'une des revendications 1 à 7, **caractérisé en ce que** la concentration stationnaire de la méthacroléine dans le réacteur est comprise entre 3 et 21 % en poids.

9. Procédé selon la revendication 8, **caractérisé en ce que** la concentration stationnaire de la méthacroléine dans le réacteur est comprise entre 5 et 20 % en poids.

10. Procédé selon au moins l'une des revendications 1 à 9, **caractérisé en ce que**, pour ce qui concerne l'alcool alkylique, il s'agit du méthanol et, pour ce qui concerne le méthacrylate d'alkyle, il s'agit du méthacrylate de méthyle.

11. Procédé selon au moins l'une des revendications 1 à 10, **caractérisé en ce que** le rapport de la masse du catalyseur au volume du mélange réactionnel liquide dans le réacteur est compris entre 0,01 et 0,3 kg/l.

12. Procédé selon au moins l'une des revendications 1 à 11, **caractérisé en ce que**, pour ce qui concerne le réacteur, il s'agit d'un réacteur à suspension.

13. Procédé selon au moins l'une des revendications 1 à 12, **caractérisé en ce que** le mélange réactionnel est prélevé en continu du réacteur et que le catalyseur reste dans le réacteur après séparation.

14. Procédé selon au moins l'une des revendications 1 à 13, **caractérisé en ce que** le mélange réactionnel, après l'extraction continue du réacteur, est traité dans au moins une colonne de distillation, l'alcool alkylique et la méthacroléine étant à cette occasion séparés sous forme d'un distillat et renvoyés dans le réacteur.
